# EUROPEAN PATENT APPLICATION

(11) **EP 3 786 109 A1**
(43) Date of publication of application: **03.03.2021**
(21) Application number: 19792871.6
(22) Date of filing: 25.04.2019
(51) Int. Cl.: C01B 25/32, A61L 27/12, A61L 27/50, C01B 25/45

(54) **HYDROXYAPATITE**

(30) Priority: 27.04.2018 JP 2018087430
(71) Applicant: Bioapatite, Inc., Hikone-city, Shiga, 522-0068 (JP)
(72) Inventor: NAKAMURA Koichi, Hikone- city, Shiga 5220068 (JP); SAKAI Yuki, Hikone- city, Shiga 5220068 (JP); KAWAMOTO Tadashi, Hikone- city, Shiga 5220068 (JP)
(74) Representative: Gill Jennings & Every LLP
(86) International application number: PCT/JP2019/017578
(87) International publication number: WO 2019/208683

(57) **Abstract**

Hydroxyapatite having high biocompatibility suitable for applications such as food additives, cosmetic ingredients, pharmaceutical ingredients, and artificial bones is provided. The hydroxyapatite of the present invention contains Mg.

## Description

### TECHNICAL FIELD

The present invention relates to hydroxyapatite having high biocompatibility.

### BACKGROUND ART

Hydroxyapatite, Ca₁₀(PO₄)₆(OH)₂, is a major component of bone and teeth. Due to its high biocompatibility, neutral pH, and high safety, hydroxyapatite is used as a biomaterial for applications including industrial materials, food additives, cosmetic ingredients, pharmaceutical ingredients, and artificial bones.

A method for producing hydroxyapatite has been proposed, in which a site for inducing precipitation of hydroxyapatite crystals is incorporated into a substrate and the substrate is immersed in an aqueous solution containing a hydroxyapatite component, to thereby precipitate hydroxyapatite crystals on the surface of the substrate (Patent document 1). There is also another method in which a substrate is coated or printed by predetermined hydroxyapatite dispersion, and then the solvent contained in the hydroxyapatite dispersion is evaporated from the substrate to thereby generate low crystalline hydroxyapatite particles on the surface of the substrate (Patent document 2).

### RELATED ART DOCUMENTS

### PATENT DOCUMENTS

Patent document 1: JP2001-031409A
Patent document 2: JP2016-147799A

### SUMMARY OF THE INVENTION

### PROBLEMS TO BE SOLVED BY THE INVENTION

As previously described, hydroxyapatite has high biocompatibility. Nevertheless, hydroxyapatite with higher biocompatibility has always been demanded in applications such as food additives, cosmetic ingredients, pharmaceutical ingredients, and artificial bones. In view of the forgoing, an object of the present invention is to provide hydroxyapatite having higher biocompatibility than conventional ones.

### MEANS FOR SOLVING THE PROBLEMS

The present inventors have conducted intensive studies to solve the above problem, and found that hydroxyapatite containing Mg shows high biocompatibility, thereby achieving the present invention.

Specifically, the present invention provides the following.
[1] Hydroxyapatite containing Mg.
[2] The hydroxyapatite according to [1], containing microcrystalline hydroxyapatite.
[3] The hydroxyapatite according to [1] or [2], represented by the following chemical formula:

   (Ca:Mg)₁₀(PO₄)₆(OH)₂

   wherein (Ca:Mg)io denotes that the total number of Ca atoms and Mg atoms equals to 10, with the number of Ca atoms being from 7 to 9, and the number of Mg atoms being from 1 to 3.
[4] The hydroxyapatite according to any one of [1] to [3], made from a biologically derived material.
[5] The hydroxyapatite according to any one of [1] to [4], further containing at least one mineral selected from Na, K, and Si.

### EFFECTS OF THE INVENTION

The hydroxyapatite of the present invention exhibits high biocompatibility by virtue of containing Mg.

### BRIEF DESCRIPTION OF THE DRAWING

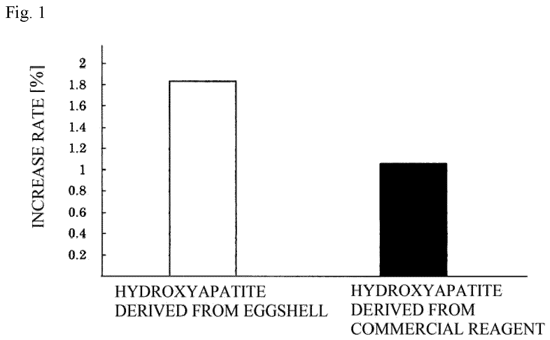

FIG.1 is a chart showing amounts of increase in a simulated body fluid of hydroxyapatite derived from eggshells in Example and hydroxyapatite in Comparative Example.

### MODE FOR CARRYING OUT THE INVENTION

The hydroxyapatite of the present invention will be described in more detail hereinbelow.

The hydroxyapatite of the present invention contains Mg (i.e., magnesium). Mg, one of the constituent minerals of natural bone, acts in natural bone to stimulate osteoblast and osteoclast activity, thereby stimulating osteocyte activity. Therefore, the hydroxyapatite of the present invention containing Mg that acts in the aforementioned manner exhibits higher biocompatibility as compared to a conventional hydroxyapatite when used as biomaterials such as food additives, cosmetic ingredients, pharmaceutical ingredients, and artificial bones.

Preferably, the content of Mg in the hydroxyapatite of the present invention is in a range of from 100 to 20.000 ppm by mass, as long as the chemical formula described hereinbelow is satisfied. When the Mg content is 100 ppm by mass or above, the effect of the incorporation of Mg becomes more prominent. The upper limit of the Mg content is not particularly limited, but as much as 20,000 ppm by mass is sufficient from the viewpoint of biocompatibility. More preferably, the Mg content is 500 to 6,000 ppm by mass.

The hydroxyapatite of the present invention preferably contains microcrystalline hydroxyapatite. The term "microcrystalline hydroxyapatite" refers to hydroxyapatite consisting exclusively of finely crystallized hydroxyapatites, or to an admixture of a finely crystallized hydroxyapatite with low crystalline hydroxyapatite having a low degree of crystallinity due to crystal distortions, crystal defects, and the like. In other words, the term "microcrystalline hydroxyapatite" as used herein is not limited to an aspect of a finely crystallized hydroxyapatite, but an aspect of a finely crystallized hydroxyapatite admixed with low crystalline hydroxyapatite is also encompassed. The low crystalline hydroxyapatite may be present in the hydroxyapatite of the present invention in a proportion of no more than 50% by mass with respect to the entire hydroxyapatite.

Hydroxyapatite containing Mg and microcrystalline hydroxyapatite shows flexible reactivity toward foreign matter, because the molecules thereof are not tightly bound to each other, but instead are only loosely gathered together. Moreover, such hydroxyapatite shows a higher adsorption capacity as compared to its crystalline form. In addition, such hydroxyapatite is less irritating by virtue of its fine particle size and smooth texture.

Identification of microcrystalline hydroxyapatite, namely, hydroxyapatite consisting exclusively of finely crystallized hydroxyapatite or an admixture of finely crystallized hydroxyapatite with low crystalline hydroxyapatite having a low degree of crystallization may be performed using X-ray structural analysis.

Specifically, when X-ray structural analysis of hydroxyapatite shows that a crystallite size determined based on the peak appeared when 2θ is from 31.500 to 32.500° is from 10 to 200Å, it may be considered that the hydroxyapatite consists exclusively of finely crystallized hydroxyapatite, or alternatively, consists of an admixture of finely crystallized hydroxyapatite with low crystalline hydroxyapatite having a low degree of crystallization.

The crystallite size as used herein refers to the size of the crystal grain, and the numerical value thereof may be used as an indicator of crystallinity. A greater numerical value of the crystallite size indicates that the analyte has a higher degree of crystallinity. In other words, a smaller numerical value of the crystallite size indicates that the analyzed hydroxyapatite has a lower degree of crystallinity or is in a finely crystallized form. The crystallite size may be determined, for example, by X-ray diffractometer Model: RINT2200V/PC from RIGAKU Corporation.

Preferably, the crystallite size determined based on the peak appeared when 2θ is from 31.500 to 32.500° is in a range of from 30 to 150Å, more preferably, in a range of from 50 to 120Å.

When the crystallite size determined by the X-ray structural analysis based on the peak appeared when 2θ is from 31.500 to 32.500° falls within the above range, the hydroxyapatite would have a surface with complex geometry and an electric charge. The hydroxyapatite with such features has a higher adsorption capacity. Thus, it may suitably be used for applications including filter materials, because such hydroxyapatite shows a superior adsorption capacity not only toward proteins and lipids but even toward bacteria and particles such as pollen particles. Furthermore, the hydroxyapatite can effectively be used for teeth whitening because it also adsorbs pigments. Additionally, hydroxyapatite having a crystallite size falling within the above range can be less irritation by virtue of its fine particle size and a smooth texture.

Preferably, the hydroxyapatite containing Mg is represented by the following chemical formula:

(Ca:Mg)₁₀(PO₄)₆(OH)₂

wherein (Ca:Mg)₁₀ denotes that the total number of Ca atoms and Mg atoms equals to 10, with the number of Ca atoms being from 7 to 9, and the number of Mg atoms being from 1 to 3. In other words, hydroxyapatite having a structure in which the constituent Ca atoms of the hydroxyapatite are partially replaced by Mg atoms is preferred.

Preferably, the hydroxyapatite of the present invention is made from a biologically derived material. The hydroxyapatite known in the related art is synthesized by various production processes using mineral-derived slaked lime as a main raw material. The hydroxyapatite synthesized from mineral-derived slaked lime as a main raw material contains almost no mineral components represented by Mg, thus exhibiting lower biocompatibility as compared to the hydroxyapatite of the present invention. In contrast, the hydroxyapatite of the present invention made from a biologically derived material can contain Mg in an appropriate amount, thus exerting the aforementioned effect of the hydroxyapatite of the present invention. The hydroxyapatite of the present invention may be produced, for example, by firing a biologically derived material to generate calcium oxide and then treating the obtained calcium oxide as described hereinbelow. The firing conditions are not particularly limited, and known conditions may be employed. However, the firing conditions may include, for example, firing by a firing means such as an electric furnace at a temperature of from 900 to 1300 °C for a duration of from 1 to 72 hours.

As the hydroxyapatite of the present invention is made from a biologically derived material, it exhibits higher safety for human bodies and is applicable to oral preparations such as calcium supplements or for food application.

Examples of the biologically derived material include eggshells and corals. Among these, eggshells are preferred because the Mg content is higher as compared to other biomaterials.

Preferably, the hydroxyapatite of the present invention further contains at least one mineral selected from Na, K, and Si. Na (i.e., sodium) is a mineral involved in bone turnover and resorption process as well as cell adhesion. K (i.e., potassium) is a mineral involved in various functions in biochemical reactions. Si (i.e., silicon) is a mineral that acts on metabolic pathways involved in osteogenesis and is involved in expression of osteocytes and connecting cells. Accordingly, if hydroxyapatite contains at least one of these minerals, the biocompatibility of the hydroxyapatite is further improved. The hydroxyapatite made from a biologically derived material contains Mg as well as at least one mineral selected from Na, K, and Si. Thus, hydroxyapatite containing Mg in an amount of 100 ppm by mass or above as well as at least one mineral selected from Na, K, and Si may be presumed to be made from the aforementioned biologically derived material.

The content of each of Na, K, and Si is not particularly limited, but for the purpose of ensuring the aforementioned effect sufficiently, it is preferable that the hydroxyapatite contains Na, K, and Si in amounts, for example, of from 100 to 5000 ppm by mass, from 10 to 100 ppm by mass, and from 10 to 100 ppm by mass, respectively. Hydroxyapatite made from a biologically derived material is a favorable material because the original mineral balance relative to the Mg content is preserved in the hydroxyapatite, and consequently, the hydroxyapatite may contain at least one mineral selected from Na, K, and Si in an amount in the above range, thus exhibiting the aforementioned effect sufficiently.

At least one mineral selected from Na, K, and Si may be incorporated in the hydroxyapatite, for example, by producing the hydroxyapatite using the aforementioned biologically derived material containing Na, K, and Si.

The method for producing the hydroxyapatite of the present invention is not particularly limited. As an illustrative example, the aforementioned biologically derived material is subjected to firing to yield calcium oxide, and the resultant calcium oxide is suspended in water or alcohol. To the resultant suspension is added a solution of phosphoric acid in water or alcohol, or alternatively, to the solution of phosphoric acid in water or an alcohol is added the suspension of the calcium oxide in water or alcohol, to thereby obtain a hydroxyapatite slurry. Subsequently, a substrate is coated or printed with the hydroxyapatite slurry, followed by evaporation of the solvent of the slurry, or alternatively, the solvent is directly evaporated from the slurry without using any substrate, to thereby obtain hydroxyapatite particles. In this way, the hydroxyapatite containing Mg may readily be obtained by employing a biologically derived material as a raw material of the calcium oxide for the calcium oxide suspension.

During the preparation of the hydroxyapatite slurry, adjustment of pH is not required. Preferably, the ratio between the total amount of the calcium oxide in the calcium oxide suspension and the total amount of the phosphoric acid in the phosphoric acid solution is adjusted such that the molar ratio of calcium ion to phosphate ion is, for example, 10:6. Needless to say, the ratio can be varied in accordance with factors such as reaction conditions. The adjustment of the molar ratio may be carried out by adjusting the concentration and the amount of the liquid to be added and the receiving liquid.

Concerning the temperature conditions during the addition of the liquid to be added to the receiving liquid, the temperatures of both liquids are preferably kept, for example, in a range from 5 to 90°C, more preferably in a range from 15 to 60°C, and still preferably in a range from 20 to 40°C. When the temperatures of these liquids are kept within the above range, the crystallization of the hydroxyapatite may be restrained as well as the reaction of hydroxyapatite formation may proceed smoothly. The liquid to be added may be added while stirring the receiving liquid.

During the evaporation of the solvent of the hydroxyapatite slurry, heating is not especially necessary. The evaporation may be performed through natural drying in an ambient temperature. However, it is possible to heat the substrate or slurry during or after the evaporation of the solvent for the purpose to enhance production efficiency and to promote lowering the degree of crystallinity of finely crystallized hydroxyapatite. The substrate is heated preferably at a temperature of between 40 to 300°C, more preferably between 40 to 180°C, and still preferably between 80 to 150°C. When the heating is performed at a temperature within the above range, it is possible to form hydroxyapatite particles with low crystallinity having appropriate particle sizes on the surface of the substrate while preventing the hydroxyapatite particles with low crystallinity from exfoliating from the surface of the substrate. The heating duration is not particularly limited, but it is sufficient to heat until hydroxyapatite particles with low crystallinity are formed. However, if the substrate after being coated or printed is excessively heated, the low crystalline hydroxyapatite might be converted to crystalline hydroxyapatite. The conversion of low crystalline hydroxyapatite to crystalline hydroxyapatite is preferably restrained because hydroxyapatite with low degree of crystallinity exhibits a greater adsorption capacity than crystalline hydroxyapatite toward finely divided substances such as biological species including bacteria and pollen as well as heavy metallic substances. Hence, as a standard of the heating conditions, for example, when heating at 100 °C or above, it is preferable that the heating time is 720 minutes or less.

### EXAMPLES

The present invention will be described in more detail hereinbelow with reference to Examples. However, not to mention, the scope of the present invention is not limited by Examples.

### Experiment 1

Hydroxyapatite samples were prepared from CaO obtained by firing eggshell as a raw material at 1000°C for 20 hours and from CaO obtained by firing coral as a raw material at 1000°C for 20 hours. In addition, a commercially available hydroxyapatite reagent was also prepared for comparative purposes. Trace elemental analysis on these hydroxyapatite samples was performed by ICP Emission Spectrometer (ICPS-8100, Shimadzu Corporation). Commercial 1000 ppm standard solutions of Mg, Na, and K from Wako Junyaku were used as reagents for the analysis. The analysis was performed on each sample solution prepared in the following manner: 1.00 g of each sample was weighted in 50 ml graduated cylinder and the sample was dissolved in a small quantity of hydrochloric acid, followed by adjusting the total volume to 50 ml.

Analytical results are shown in Table 1. Numerical values in Table 1 are expressed in ppm by mass (mg/kg).

**[Table 1]**

| | Hydroxyapatite derived from eggshell | Hydroxyapatite derived from coral | Hydroxyapatite manufactured by Wako Junyaku |
|---|---|---|---|
| Mg | 2039 | 1675 | >10 |
| Na | 352 | 3850 | ND |
| K | 68 | 22 | ND |
| Si | 45 | 550 | ND |
| Fe | 21 | 210 | ND |

As can be seen from Table 1, the hydroxyapatite prepared from the biologically derived raw material contains Mg. Especially, the hydroxyapatite derived from eggshell contains a larger amount of Mg. This Mg content is closer to an exemplary Mg content of human bone, 5500 ppm, thus suggesting that the hydroxyapatite derived from eggshell may have a higher biocompatibility.

### Experiment 2

Bioactivity of the hydroxyapatite derived from eggshell was evaluated by using simulated body fluid (SBF). In this evaluation, a sample was immersed in simulated body fluid (SBF), and after a predetermined period of time, the amount of hydroxyapatite grown on the sample was determined. The simulated body fluid herein contains inorganic ions at concentrations nearly identical to those of human body fluid, and was prepared with the following ingredients.

NaCl: 7.996 g, NaHCO₃: 0.350 g, KCl: 0.224 g, K₂HPO₄ and 3H₂O: 0.228 g, MgCl₂ and 6H₂O: 0.350 g, 1M HCl: 40 ml, CaCl₂ and 2H₂O: 0.278 g, NaSO₄: 0.071 g, Tris (Buffer /tris(hydroxyethyl)aminomethane): 6.057 g. These reagents were added to 700 ml of distilled water to adjust the pH to 7.4, and then distilled water was added to bring a total volume of 1000 ml.

For the evaluation of bioactivity of hydroxyapatite, two types of slurries were prepared: a slurry of hydroxyapatite (hydroxyapatite A, Mg content: 1974 ppm) prepared from CaO obtained by firing eggshell as a raw material at 1000°C for 20 hours, and a slurry of hydroxyapatite (hydroxyapatite B, no Mg content) prepared from CaO obtained by firing a commercial Ca(OH)₂ reagent (available from Wako Junyaku, purity: 99%) as a raw material at 1000°C for 20 hours.

A slurry containing hydroxyapatite A or hydroxyapatite B was applied onto a felt cloth with dimension: 1 cm × 5 cm (six different clothes were used for each slurry), and the resultant cloth was dried at 120°C for 2 hours, to thereby form hydroxyapatite particles on the cloth as a sample. Each of the felt clothes was weighed before and after the formation of the hydroxyapatite particles, and the mass of the hydroxyapatite particles formed was calculated.

Each of the samples was immersed separately from one another in 50 ml of simulated body fluid and left at 37°C for 7 days. Subsequently, the samples were taken out from simulated body fluid, dried at 130°C for 2 hours, and then kept in a desiccator.

Thereafter, each sample was weighed and the mass gain of hydroxyapatite was calculated.

The result of the measurement on the hydroxyapatite A is shown in Table 2.

**[Table 2]**

| | before immersion | after immersion | amount increased |
|---|---|---|---|
| No.1 | 0.2300 | 0.2353 | 0.0053 |
| No.2 | 0.2793 | 0.2840 | 0.0047 |
| No.3 | 0.3003 | 0.3064 | 0.0061 |
| No.4 | 0.2873 | 0.2924 | 0.0051 |
| No.5 | 0.3244 | 0.3295 | 0.0051 |
| No.6 | 0.3081 | 0.3132 | 0.0051 |
| average | 0.2882 | 0.2935 | 0.0052 |

The result of the measurement on the hydroxyapatite B is shown in Table 3.

**[Table 3]**

| | before immersion | after immersion | amount increased |
|---|---|---|---|
| No.1 | 0.2696 | 0.2714 | 0.0018 |
| No.2 | 0.2580 | 0.2618 | 0.0038 |
| No.3 | 0.3158 | 0.3193 | 0.0035 |
| No.4 | 0.2813 | 0.2842 | 0.0029 |
| No.5 | 0.3330 | 0.3335 | 0.0025 |
| No.6 | 0.2937 | 0.2978 | 0.0041 |
| average | 0.2919 | 0.2950 | 0.0031 |

The percentage of mass increase of hydroxyapatite after immersion with respect to the mass before immersion is defined as "increase rate". The average increase rate of the hydroxyapatite A derived from eggshell as shown in Table 2 was 1.84%, whereas the average increase rate of the hydroxyapatite B as shown in Table 3 was 1.06%, showing that a larger amount of hydroxyapatite was formed in simulated body fluid with the hydroxyapatite A as compared to the hydroxyapatite B. This means that biocompatibility of the hydroxyapatite A containing Mg is higher than the hydroxyapatite B that does not contain Mg.

The results shown in Tables 2 and 3 are also shown in the chart in FIG. 1.

## Claims

1. Hydroxyapatite comprising Mg.

2. The hydroxyapatite according to claim 1, comprising microcrystalline hydroxyapatite.

3. The hydroxyapatite according to claim 1 or 2, represented by the following chemical formula:
(Ca:Mg)₁₀(PO₄)₆(OH)₂
wherein (Ca:Mg)₁₀ denotes that the total number of Ca atoms and Mg atoms equals to 10, with the number of Ca atoms being from 7 to 9, and the number of Mg atoms being from 1 to 3.

4. The hydroxyapatite according to any one of claims 1 to 3, made from a biologically derived material.

5. The hydroxyapatite according to any one of claims 1 to 4, further comprising at least one mineral selected from Na, K, and Si.
